# EUROPEAN PATENT APPLICATION

(11) **EP 3 047 857 A1**
(43) Date of publication of application: **27.07.2016**
(21) Application number: 14845545.4
(22) Date of filing: 19.09.2014
(51) Int. Cl.: A61K 39/395, A61K 45/00, A61P 7/04, C07K 16/18

(54) **TREATMENT FOR HEMORRHAGIC DISEASES BY ANTI-PROTEIN-C ANTIBODY**

(30) Priority: 20.09.2013 JP 2013195355
(71) Applicant: Chugai Seiyaku Kabushiki Kaisha, Kita-ku Tokyo 115-8543 (JP)
(72) Inventor: KITAZAWA, Takehisa, Gotemba-shi Shizuoka 412-8513 (JP); NAKANE, Aya, Gotemba-shi Shizuoka 412-8513 (JP); YOSHIHASHI, Kazutaka, Kamakura-shi Kanagawa 247-8530 (JP); SOEDA, Tetsuhiro, Gotemba-shi Shizuoka 412-8513 (JP); MUTO, Atsushi, Gotemba-shi Shizuoka 412-8513 (JP)
(74) Representative: Vossius & Partner Patentanwälte Rechtsanwälte mbB
(86) International application number: PCT/JP2014/074789
(87) International publication number: WO 2015/041310

(57) **Abstract**

The present inventors have completed the present invention by finding that an excellent anticoagulation inhibitory effect is obtained by the administration of an agent inhibiting the activation of protein C.

## Description

### Technical Field

The present invention relates to a composition for the treatment of a hemorrhagic disease, comprising an agent inhibiting the activation of protein C. Specifically, the present invention relates to a composition comprising an anti-protein C antibody inhibiting the activation of protein C as an active ingredient.

### Background Art

Hemophilia A is a hemorrhagic disease caused by congenital deficiency or dysfunction of blood coagulation factor VIII (FVIII) or blood coagulation factor IX (FIX). The former disease is called hemophilia A, and the latter disease is called hemophilia B. Both genes are located on the X chromosome. Since gene abnormality is transmitted by sex-linked recessive inheritance, 99% or more of patients who develop this disease are males. It is known that the prevalence of hemophilia is about 1 in 10,000 births and that the ratio between hemophilia A and hemophilia B is about 5:1.

Examples of principal sites of bleeding include sites in joints, in muscles, under the skin, in the mouth, in the cranium, in the gastrointestinal tract, and in the nasal cavity. Among them, repetitive bleeding in joints progresses to joint disorder or hemophilic arthropathy with walking difficulty and may eventually require joint replacement. This is therefore largely responsible for reduction in QOL of hemophilia patients.

The severity of hemophilia depends on the activity of FVIII or the activity of FIX in blood and is classified into severe patients with less than 1% activity; moderate patients with 1% or more and less than 5% activity; and mild patients with 5% or more and less than 40% activity. Severe patients who account for approximately 60% of hemophilia patients manifest a bleeding symptom a couple of times a month. This is much more frequent as compared with moderate patients and mild patients. This suggests that severe hemophilia patients are effectively prevented from manifesting a bleeding symptom by maintaining 1% or more activity of FVIII or FIX in blood (Non Patent Literature 1).

In addition to hemophilia and acquired hemophilia, von Willebrand disease caused by functional abnormality or deficiency in vWF is known as related abnormal bleeding. The von Willebrand factor (vWF) is not only necessary for platelet to adhere normally to subendothelial tissues in the injury site of a vascular wall but necessary for forming a complex with FVIII and keeping FVIII levels in plasma at normal levels. In von Willebrand disease patients, these functions decline and incur the functional abnormality of hemostasis.

Blood coagulation factors purified from plasma or prepared by a gene recombination technique are mainly used in the prevention and/or treatment of bleeding in hemophilia patients.

The blood coagulation factors are not sufficiently sustained (their half-lives are several hours to several tens of hours). For example, the half-lives of an FVIII preparation and an FIX preparation in blood are on the order of 12 hours and 24 hours, respectively. Hence, continuous prevention requires administering the FVIII preparation approximately three times a week or the FIX preparation approximately twice a week. This corresponds to the maintenance of about 1% or more FVIII activity or FIX activity. This preventive administration can prevent the occurrence of hemophilic joint disorder caused by frequent intraarticular bleeding and, reportedly, consequently contributes largely to improvement in QOL of hemophilia A patients.

Replacement therapy in the event of bleeding, except for mild bleeding, also requires additionally administering the FVIII preparation or the FIX preparation on a regular basis for a given period for preventing rebleeding and achieving complete hemostasis.

The blood coagulation factors further have the disadvantage that intravenous administration is necessary. Technical difficulty is found in the intravenous administration of the FVIII preparation and the FIX preparation. In particular, administration to young patients is more difficult because the vein used in the administration is thin.

In many cases, domestic therapy or self injection is used in the preventive administration mentioned above or emergent administration in the event of bleeding. The need of frequent administration and technical difficulty in administration do not only suffer recipient patients but are responsible for a hindrance to the widespread use of domestic therapy or self injection.

Thus, there has been a strong demand for a drug with wider dosing intervals or a drug that is administered more easily as compared with the existing coagulation factor preparations.

An antibody against FVIII or FIX, called inhibitor, may further emerge in hemophilia patients, particularly, severe hemophilia patients. Once the inhibitor emerges, the effect of a coagulation factor preparation is hindered by the inhibitor. As a result, neutralization therapy using a large amount of the coagulation factor preparation or bypass therapy using a complex concentrate or activated blood coagulation factor VII (FVIIa preparation) is practiced. In either case, however, the control of hemostasis for patients is very difficult on the ground that, for example, regular replacement prevention has not been established.

Thus, there has been a strong demand for a drug that is independent of the presence of such an inhibitor.

Antibodies have received attention and have been applied as drugs, because of being highly stable in blood, being subcutaneously administrable, and also having low antigenicity. The antibodies seem to be useful for the development of drugs on the grounds of (i) wide dosing intervals, (ii) easy administration, and (iii) independence of the presence of inhibitors. The half-lives of the antibodies in blood are generally relatively long and are several days to several weeks. Also, the antibodies are generally known to migrate into blood after subcutaneous administration. In addition, the antibodies differ largely in structure from FVIII or FIX and have low antigenicity. This means that the antibody drugs satisfy the conditions (i), (ii), and (iii) described above.

Meanwhile, protein C (PC) is a factor that works in the negative feedback mechanism in blood coagulation. PC binds to endothelial protein C receptor (EPCR) expressed in the vascular endothelium and becomes activated protein C (APC) when activated by a thrombin/thrombomodulin complex. APC, together with its cofactor protein S, inactivates activated blood coagulation factor VIII (FVIIIa) and activated blood coagulation factor V (FVa) (Non Patent Literature 2). Accordingly, it is easy to assume that the inhibition of PC is useful in the promotion of coagulation. In fact, patients with homozygous congenital protein C deficiency manifest thrombotic fulminant purpura immediately after birth. According to another report, the amount of a coagulation factor preparation used is small for patients having a gene mutation (FV Leiden) that imparts APC resistance to blood coagulation factor V (FV), among severe hemophilia A patients. These reports support this assumption (Non Patent Literature 3). In animal experiments, Schlachterman et al. have showed that when the FV Leiden mutation is introduced to an FVIII- or FIX-deficient mouse, coagulation is promoted in microcirculation by particular stimulation (Non Patent Literature 4). Butenas et al. have developed an agent inhibiting APC and showed that this agent partially restores reduction in thrombin generation caused by FVIII deficiency in a synthetic coagulation proteome model (Non Patent Literature 5).

The possibility has also been reported that an antibody inhibiting the activity of APC exhibits a blood-clotting effect and can be used in the treatment of hemophilia (Patent Literature 1).

Nonetheless, a blood-clotting effect brought about by the *in vivo* inhibition of APC activity has not yet been confirmed. The paper of Schlachterman et al. mentioned above gives the inconsistent results showing that FV Leiden has no hemostatic effect on an FVIII-deficient mouse, but has a hemostatic effect on an FXI-deficient mouse.

Information on the prior technical literatures related to the invention of the present application is given below.

### Citation List

### Non Patent Literature

Non Patent Literature 1: Astermark J. Haemophilia. 2003, 9 (Suppl. 1), 32
Non Patent Literature 2: Castellino FJ and Ploplis VA, J Thromb and Haemost, 2009, 7, (Suppl. 1), 140
Non Patent Literature 3: van Dijk K et al., J Thromb and Haemost, 2004, 92, 305
Non Patent Literature 4: Schlachterman A et al., J Thromb Haemost 2005, 3, 2730
Non Patent Literature 5: Butenas S et al., J Thromb Haemost 2006, 4, 2411

### Patent Literature

Patent Literature 1: National Publication of International Patent Application No. 2011-500843

### Summary of Invention

### Technical Problem

An object of the present invention is to provide a novel composition for the treatment of a hemorrhagic disease having an excellent blood-clotting effect, a composition for the inhibition of an anti-clotting effect, or a composition for the promotion of a hemostatic effect.

### Solution to Problem

The present inventors have conducted diligent studies to attain the object and consequently completed the present invention by finding that a high hemostatic effect is obtained by the inhibition of the activation of protein C.

More specifically, the present invention provides the following [1] to [14]:
[1] A pharmaceutical composition for the treatment of a hemorrhagic disease, comprising an agent inhibiting the activation of protein C.
[2] The composition according to [1], wherein the hemorrhagic disease is a disease selected from hemophilia, acquired hemophilia, von Willebrand disease caused by functional abnormality or deficiency in vWF, and acquired von Willebrand disease.
[3] The composition according to [1], wherein the hemorrhagic disease is hemophilia A.
[4] A pharmaceutical composition for the promotion of a hemostatic effect, comprising an agent inhibiting the activation of protein C.
[5] The composition according to [4], wherein the hemostatic effect is an effect on the bleeding symptom of a disease selected from hemophilia, acquired hemophilia, von Willebrand disease caused by functional abnormality or deficiency in vWF, and acquired von Willebrand disease.
[6] The composition according to [4], wherein the hemostatic effect is an effect on the bleeding symptom of hemophilia A.
[7] A pharmaceutical composition for the inhibition of an anticoagulant effect, comprising an agent inhibiting the activation of protein C.
[8] The composition according to [7], wherein the anticoagulant effect is the anticoagulant effect of activated protein C.
[9] The composition according to any one of [1] to [8], wherein the agent inhibiting the activation of protein C further inhibits the activity of the activated protein C.
[10] The composition according to any one of [1] to [9], wherein the pharmaceutical composition is a composition that is used in combination with an agent inhibiting the activity of the activated protein C.
[11] The composition according to any one of [1] to [10], wherein the agent inhibiting the activation of protein C is an anti-protein C antibody.
[12] The composition according to [11], wherein the anti-protein C antibody is an antibody binding to the heavy chain of the protein C.
[13] An anti-protein C antibody having an effect of inhibiting the conversion of protein C to activated protein C by binding to the heavy chain of the protein C.
[14] The antibody according to [13], wherein the anti-protein C antibody further has an effect of inhibiting the activity of the activated protein C.
[15] A method for treating a hemorrhagic disease, comprising the step of administering an agent inhibiting the activation of protein C.
[16] The method according to [15], wherein the hemorrhagic disease is a disease selected from hemophilia, acquired hemophilia, von Willebrand disease caused by functional abnormality or deficiency in vWF, and acquired von Willebrand disease.
[17] The method according to [15], wherein the hemorrhagic disease is hemophilia A.
[18] A method for promoting a hemostatic effect, comprising the step of administering an agent inhibiting the activation of protein C.
[19] The method according to [18], wherein the hemostatic effect is an effect on the bleeding symptom of a disease selected from hemophilia, acquired hemophilia, von Willebrand disease caused by functional abnormality or deficiency in vWF, and acquired von Willebrand disease.
[20] The method according to [18], wherein the hemostatic effect is an effect on the bleeding symptom of hemophilia A.
[21] A method for inhibiting an anticoagulant effect, comprising the step of administering an agent inhibiting the activation of protein C.
[22] The method according to [21], wherein the anticoagulant effect is the anticoagulant effect of activated protein C.
[23] The method according to any of [15] to [22], wherein the agent inhibiting the activation of protein C further inhibits the activity of the activated protein C.
[24] The method according to any of [15] to [23], wherein the agent inhibiting the activation of protein C is administered in combination with an agent inhibiting the activity of the activated protein C.
[25] The method according to any of [15] to [24], wherein the agent inhibiting the activation of protein C is an anti-protein C antibody.
[26] The method according to [25], wherein the anti-protein C antibody is an antibody binding to the heavy chain of the protein C.
[27] Use of an agent inhibiting the activation of protein C for producing a drug for the treatment of a hemorrhagic disease.
[28] The use according to [27], wherein the hemorrhagic disease is a disease selected from hemophilia, acquired hemophilia, von Willebrand disease caused by functional abnormality or deficiency in vWF, and acquired von Willebrand disease.
[29] The use according to [27], wherein the hemorrhagic disease is hemophilia A.
[30] Use of an agent inhibiting the activation of protein C for producing a drug for the promotion of a hemostatic effect.
[31] The use according to [30], wherein the hemostatic effect is an effect on the bleeding symptom of a disease selected from hemophilia, acquired hemophilia, von Willebrand disease caused by functional abnormality or deficiency in vWF, and acquired von Willebrand disease.
[32] The use according to [30], wherein the hemostatic effect is an effect on the bleeding symptom of hemophilia A.
[33] Use of an agent inhibiting the activation of protein C for producing a drug for the inhibition of an anticoagulant effect.
[34] The use according to [33], wherein the anticoagulant effect is the anticoagulant effect of activated protein C.
[35] The use according to any of [27] to [34], wherein the agent inhibiting the activation of protein C further inhibits the activity of the activated protein C.
[36] The use according to any of [27] to [35], wherein the drug further comprises an agent inhibiting the activity of the activated protein C.
[37] The use according to any of [27] to [35], wherein the agent inhibiting the activation of protein C is an anti-protein C antibody.
[38] The use according to [37], wherein the anti-protein C antibody is an antibody binding to the heavy chain of the protein C.
[39] An agent inhibiting the activation of protein C, the agent being used for treating a hemorrhagic disease.
[40] The agent according to [39], wherein the hemorrhagic disease is a disease selected from hemophilia, acquired hemophilia, von Willebrand disease caused by functional abnormality or deficiency in vWF, and acquired von Willebrand disease.
[41] The agent according to [39], wherein the hemorrhagic disease is hemophilia A.
[42] An agent inhibiting the activation of protein C, the agent being used for promoting a hemostatic effect.
[43] The agent according to [42], wherein the hemostatic effect is an effect on the bleeding symptom of a disease selected from hemophilia, acquired hemophilia, von Willebrand disease caused by functional abnormality or deficiency in vWF, and acquired von Willebrand disease.
[44] The agent according to [42], wherein the hemostatic effect is an effect on the bleeding symptom of hemophilia A.
[45] An agent inhibiting the activation of protein C, the agent being used for inhibiting an anticoagulant effect.
[46] The agent according to [45], wherein the anticoagulant effect is the anticoagulant effect of activated protein C.
[47] The agent according to any of [39] to [46], wherein the agent inhibiting the activation of protein C further inhibits the activity of the activated protein C.
[48] The agent according to any of [39] to [47], wherein the agent is used in combination with an agent inhibiting the activity of the activated protein C.
[49] The agent according to any of [39] to [48], wherein the agent inhibiting the activation of protein C is an anti-protein C antibody.
[50] The agent according to [49], wherein the anti-protein C antibody is an antibody binding to the heavy chain of the protein C.

### Brief Description of Drawings

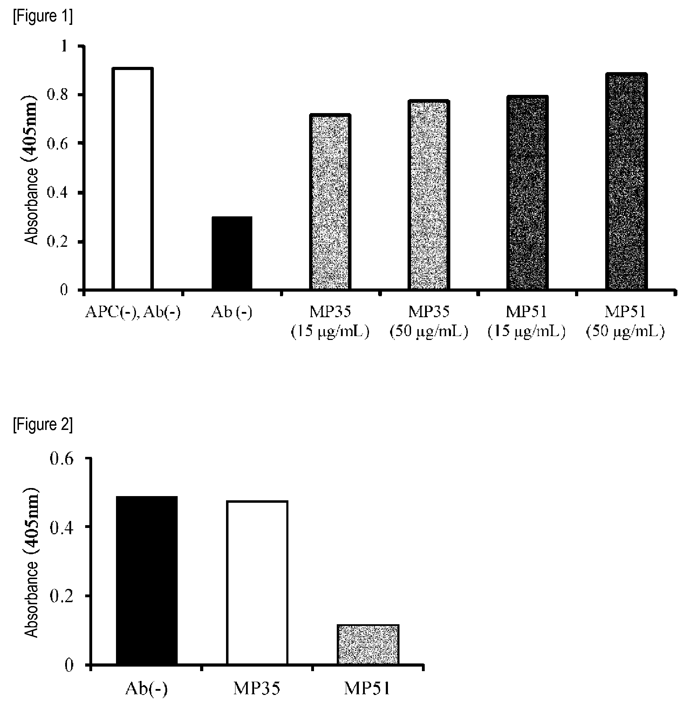

[Figure 1] Figure 1 is a diagram showing the effect of each anti-mouse protein C antibody on the inactivation of FVa by mouse activated protein C. The absorbance on the y-axis depicts the activity of generated thrombin, and a larger amount of residual FVa results in higher absorbance. A group supplemented with neither the mouse activated protein C nor the antibody is indicated by APC(-), Ab(-), and the other groups represent groups supplemented with the mouse activated protein C. As a result of the experiment, the absorbance was elevated by the addition of MP35 and MP51 compared with no addition of the antibody, demonstrating that MP35 and MP51 increase residual FVa, i.e., have inhibitory activity against the inactivation of FVa by activated protein C.
[Figure 2] Figure 2 is a diagram showing the effect of each anti-protein C antibody on the activation of mouse protein C by thrombin/thrombomodulin. The antibody concentration in mouse protein C activation reaction was 30 µg/mL. The absorbance value on the y-axis depicts the activity of the resulting mouse activated protein C. The inhibitory activity against the activation of mouse protein C by thrombin/thrombomodulin is strong. As a result of the experiment, the absorbance was decreased by the addition of MP51 compared with no addition of the antibody, demonstrating that MP51 has inhibitory activity against the activation of mouse protein C by thrombin/thrombomodulin. By contrast, the absorbance was not decreased by the addition of MP35 compared with no addition of the antibody, demonstrating that MP35 lacks inhibitory activity against the activation of mouse protein C by thrombin/thrombomodulin.

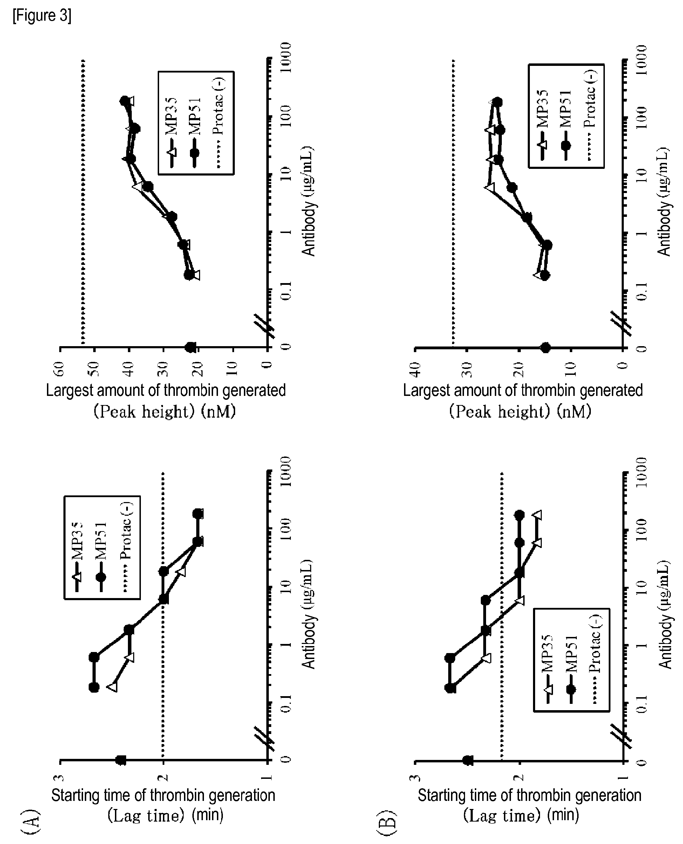

[Figure 3] Figure 3 is a diagram showing the effect of each anti-mouse protein C antibody on thrombin generation in normal mouse plasma (A) or FVIII-deficient mouse plasma (B) supplemented with an agent activating protein C. The strength of the anti-clotting effect of activated protein C generated by the addition of Protac as the agent activating protein C is indicated by lag time and peak height. The concentration of the added antibody solution was 0, 0.18, 0.6, 1.8, 6, 18, 60, and 180 µg/mL. The broken line depicts data on plasma supplemented with neither the agent activating protein C (Protac) nor the antibody. A shorter lag time or a larger peak height on the ordinate than that of an antibody-nonsupplemented group (0 µg/mL) means stronger inhibitory activity against the anti-clotting effect of activated protein C. As a result, both of MP35 (Δ) and MP51 (•) in normal mouse plasma or FVIII-deficient mouse plasma shortened the lag time in a concentration-dependent manner in the presence of the protein C activator and increased the peak height in a concentration-dependent manner in the presence of the protein C activator, demonstrating that MP35 and MP51 have the activity of inhibiting the anti-clotting effect of activated protein C.

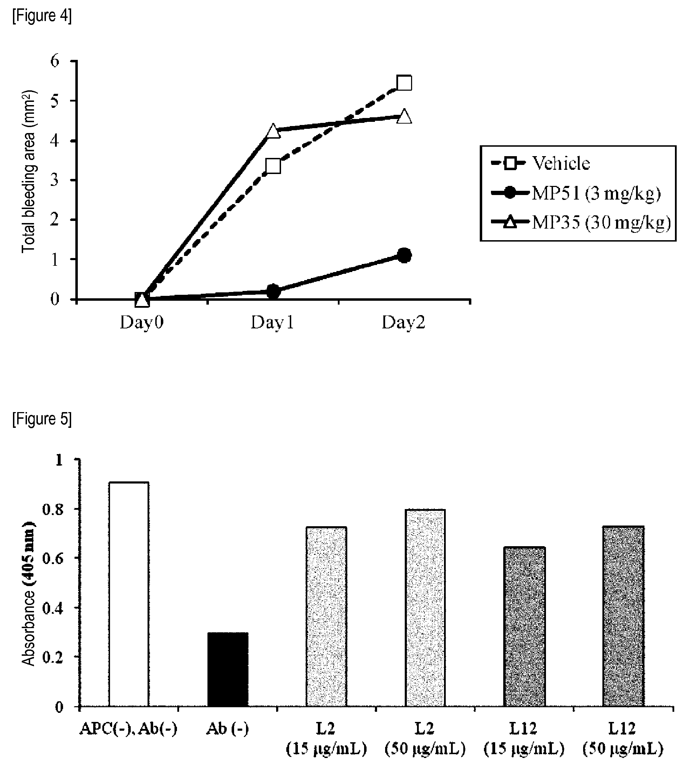

[Figure 4] Figure 4 is a diagram showing the effect of each anti-mouse protein C antibody on a bleeding symptom in a puncture bleeding model using a hemophilia A mouse. The intensity of the bleeding symptom caused by puncture is indicated by total bleeding area, and each data represents an average value of each group. A smaller total bleeding area on the ordinate than that of the vehicle group (0 )µg/mL) means a stronger hemostatic effect on the bleeding symptom caused by puncture. As a result, the total bleeding area was reduced by the administration of MP51, demonstrating that MP51 has a hemostatic effect. MP35 exhibited no hemostatic effect.
[Figure 5] Figure 5 is a diagram showing the effect of each anti-mouse protein C antibody on the inactivation of FVa by mouse activated protein C. The absorbance on the y-axis depicts the activity of generated thrombin, and a larger amount of residual FVa results in higher absorbance. A group supplemented with neither the mouse activated protein C nor the antibody is indicated by APC(-), Ab(-), and the other groups represent groups supplemented with the mouse activated protein C. The antibody concentration was 15 or 50 µg/mL. As a result of the experiment, the absorbance was elevated by the addition of L2 and L12 compared with no addition of the antibody, demonstrating that L2 and L12 increase residual FVa, i.e., have inhibitory activity against the inactivation of FVa by activated protein C.

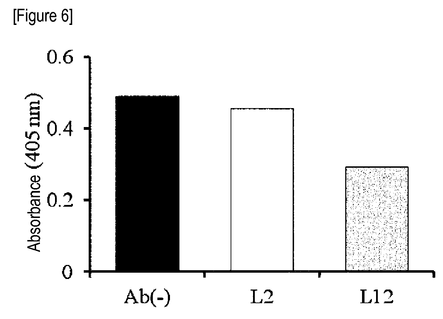

[Figure 6] Figure 6 is a diagram showing the effect of each anti-protein C antibody on the activation of mouse protein C by thrombin/thrombomodulin. The antibody concentration in mouse protein C activation reaction was 30 µg/mL. The absorbance value on the y-axis depicts the activity of the resulting mouse activated protein C. The inhibitory activity against the activation of mouse protein C by thrombin/thrombomodulin is strong. As a result of the experiment, the absorbance was decreased by the addition of L12 compared with no addition of the antibody, demonstrating that L12 has inhibitory activity against the activation of mouse protein C by thrombin/thrombomodulin. By contrast, the absorbance was not decreased by the addition of L2 compared with no addition of the antibody, demonstrating that L2 lacks inhibitory activity against the activation of mouse protein C by thrombin/thrombomodulin.

### Description of Embodiments

The present invention provides a pharmaceutical composition for the treatment of a hemorrhagic disease, comprising an agent inhibiting the activation of protein C.

In the present invention, the phrase "inhibition of the activation of protein C", "inhibiting the activation of protein C", or "inhibiting the conversion of protein C to activated protein C" means to inhibit the degradation of protein C by thrombin that has formed a complex with thrombomodulin. Examples of the agent inhibiting the activation of protein C include agents inhibiting the binding between protein C and thrombin. Specific examples thereof include anti-protein C antibodies, anti-thrombin antibodies, anti-thrombomodulin antibodies, partial peptides of protein C, partial peptides of thrombin, partial peptides of thrombomodulin, and low-molecular compounds that exhibit similar activity thereto.

In a preferred embodiment, the agent inhibiting the activation of protein C can be an anti-protein C antibody.

In the present specification, the antibody refers to a natural immunoglobulin or immunoglobulin produced by partial or complete synthesis. The antibody may be isolated from a natural resource (e.g., plasma or serum containing naturally occurring antibodies) or the culture supernatant of antibody-producing hybridoma cells or may be partially or completely synthesized by use of an approach such as gene recombination. Preferred examples of the antibody include isotypes of immunoglobulins and subclasses of these isotypes. Nine types of classes (isotypes), i.e., IgG1, IgG2, IgG3, IgG4, IgA1, IgA2, IgD, IgE, and IgM, are known as human immunoglobulins. Of these isotypes, IgG1, IgG2, IgG3, or IgG4 is preferred for the antibody of the present invention.

A method for preparing an antibody having desired binding activity is known to those skilled in the art. Examples of the method include, but are not limited to, methods for preparing an antibody binding to protein C.

The anti-protein C antibody can be obtained as a polyclonal or monoclonal antibody using means known in the art. A mammal-derived monoclonal antibody can be preferably prepared as the antibody. The mammal-derived monoclonal antibody encompasses, for example, those produced by hybridomas and those produced by host cells transformed with expression vectors containing an antibody gene by a genetic engineering approach. The monoclonal antibody of the present invention includes a "humanized antibody" and a "chimeric antibody".

The monoclonal antibody-producing hybridomas can be prepared by use of a technique known in the art, for example, as follows: mammals are immunized with protein C used as a sensitizing antigen according to a usual immunization method. Immunocytes thus obtained are fused with parental cells known in the art by a usual cell fusion method. Next, cells producing a monoclonal antibody binding to an epitope in the protein C molecule can be screened for by a usual screening method to select hybridomas producing the anti-protein C antibody.

The monoclonal antibody is prepared, for example, as follows: first, a gene sequence encoding protein C is inserted into expression vectors known in the art, with which appropriate host cells are then transformed. The desired protein C is purified from the host cells or from a culture supernatant thereof by a method known in the art.

This purified protein C can be used as the sensitizing antigen for use in the immunization of mammals. A partial peptide of protein C can also be used as the sensitizing antigen. This partial peptide may be obtained by chemical synthesis from the amino acid sequence of protein C. Alternatively, the partial peptide may be obtained by the incorporation of a portion of the protein C gene into expression vectors followed by its expression. Furthermore, the partial peptide can also be obtained by the degradation of protein C with a proteolytic enzyme. The region and size of the protein C peptide for use as such a partial peptide are not particularly limited by specific embodiments.

Examples of a preferred binding region for the anti-protein C antibody to inhibit the activation of protein C include the heavy chain of the protein C. Particularly, a moiety involved in binding to thrombin is preferred. Specifically, an activation peptide in the heavy chain or an epitope present in the neighborhood thereof is preferred. In this context, the epitope present in the neighborhood means an epitope located in a region where the anti-protein C antibody can conformationally inhibit the binding of protein C to thrombin when binding to the protein C. Such an antibody is capable of inhibiting the conversion of protein C to activated protein C. Thus, in the present invention, an arbitrary sequence is selected from an amino acid sequence corresponding to the heavy chain of the protein C and preferably used as the sensitizing antigen. The number of amino acids constituting the peptide used as the sensitizing antigen is preferably at least 5 or more, for example, 6 or more or 7 or more. More specifically, a peptide of 8 to 50, preferably 10 to 30 residues can be used as the sensitizing antigen.

Also, a fusion protein comprising a desired partial polypeptide or peptide of the protein C fused with a different polypeptide can be used as the sensitizing antigen. For example, an antibody Fc fragment or a peptide tag can be preferably used for producing the fusion protein for use as the sensitizing antigen. Two or more types of genes respectively encoding the desired polypeptide fragments are fused in frame, and the fusion gene can be inserted into expression vectors as described above to prepare vectors for the expression of the fusion protein. The method for preparing the fusion protein is described in Molecular Cloning 2nd ed. (Sambrook, J. et al., Molecular Cloning 2nd ed., 9.47-9.58 (1989), Cold Spring Harbor Lab. Press).

The mammals to be immunized with the sensitizing antigen are not limited to specific animals. The mammals to be immunized are preferably selected in consideration of compatibility with the parental cells for use in cell fusion. In general, rodents (e.g., mice, rats, and hamsters), rabbits, monkeys, or the like are preferably used.

These animals are immunized with the sensitizing antigen according to a method known in the art. For example, a general immunization method involves administering the sensitizing antigen to the mammals by intraperitoneal or subcutaneous injection. Specifically, the sensitizing antigen diluted with PBS (phosphate-buffered saline), saline, or the like at an appropriate dilution ratio is mixed with a usual adjuvant, for example, a Freund's complete adjuvant, if desired, and emulsified. Then, the resulting sensitizing antigen is administered to the mammals several times at 4- to 21-day intervals. Also, an appropriate carrier may be used in the immunization with the sensitizing antigen. Particularly, in the case of using a partial peptide having a small molecular weight as the sensitizing antigen, immunization with the sensitizing antigen peptide bound with a carrier protein such as albumin or keyhole limpet hemocyanin may be desirable in some cases.

Alternatively, the hybridomas producing the desired antibody can also be prepared as described below by use of DNA immunization. The DNA immunization is an immunization method which involves immunostimulating immunized animals by expressing *in vivo* the sensitizing antigen in the immunized animals given vector DNAs that have been constructed in a form capable of expressing the antigenic protein-encoding gene in the immunized animals. The DNA immunization can be expected to be superior to the general immunization method using the administration of the protein antigen to animals to be immunized as follows:
- the DNA immunization can provide immunostimulation with the protein structure maintained; and
- the DNA immunization eliminates the need of purifying the immunizing antigen.

In order to obtain the monoclonal antibody of the present invention by the DNA immunization, first, a DNA encoding protein C is administered to the animals to be immunized. The DNA encoding protein C can be synthesized by a method known in the art such as PCR. The obtained DNA is inserted into appropriate expression vectors, which are then administered to the animals to be immunized. For example, commercially available expression vectors such as pcDNA3.1 can be preferably used as the expression vectors. A method generally used can be used as a method for administering the vectors to the organisms. For example, gold particles with the expression vectors adsorbed thereon can be transferred into the cells of animal individuals to be immunized using a gene gun to thereby perform the DNA immunization.

A rise in the titer of the antibody binding to protein C is confirmed in the serum of the mammals thus immunized. Then, immunocytes are collected from the mammals and subjected to cell fusion. Particularly, spleen cells can be used as preferred immunocytes.

Mammalian myeloma cells are used in the cell fusion with the immunocytes. The myeloma cells preferably have an appropriate selection marker for screening. The selection marker refers to a character that can survive (or cannot survive) under particular culture conditions. For example, hypoxanthine-guanine phosphoribosyltransferase deficiency (hereinafter, abbreviated to HGPRT deficiency) or thymidine kinase deficiency (hereinafter, abbreviated to TK deficiency) is known in the art as the selection marker. Cells having the HGPRT or TK deficiency is sensitive to hypoxanthine-aminopterin-thymidine (hereinafter, abbreviated to HAT-sensitive). The HAT-sensitive cells are killed in a HAT selective medium because the cells fail to synthesize DNA. By contrast, these cells, when fused with normal cells, become able to grow even in the HAT selective medium because the fused cells can continue DNA synthesis through the use of the salvage pathway of the normal cells.

The cells having the HGPRT or TK deficiency can be selected in a medium containing 6-thioguanine or 8-azaguanine for the HGPRT deficiency or 5'-bromodeoxyuridine for the TK deficiency. The normal cells are killed by incorporating these pyrimidine analogs into their DNAs. By contrast, the cells deficient in these enzymes can survive in the selective medium because the cells cannot incorporate the pyrimidine analogs therein. In addition, a selection marker called G418 resistance confers resistance to a 2-deoxystreptamine antibiotic (gentamicin analog) through a neomycin resistance gene. Various myeloma cells suitable for cell fusion are known in the art.

For example, P3 (P3x63Ag8.653) (J. Immunol. (1979) 123 (4), 1548-1550), P3x63Ag8U.1 (Current Topics in Microbiology and Immunology (1978) 81, 1-7), NS-1 (C. Eur. J. Immunol. (1976) 6 (7), 511-519), MPC-11 (Cell (1976) 8 (3), 405-415), SP2/0 (Nature (1978) 276 (5685), 269-270), FO (J. Immunol. Methods (1980) 35 (1-2), 1-21), S194/5.XX0.BU.1 (J. Exp. Med. (1978) 148 (1), 313-323), or R210 (Nature (1979) 277 (5692), 131-133) can be preferably used as such myeloma cells.

Basically, the cell fusion of the immunocytes with the myeloma cells is carried out according to a method known in the art, for example, the method of Kohler and Milstein et al. (Methods Enzymol. (1981) 73, 3-46).

More specifically, the cell fusion can be carried out, for example, in a usual nutrient medium in the presence of a cell fusion promoter. For example, polyethylene glycol (PEG) or hemagglutinating virus of Japan (HVJ) is used as the fusion promoter. In addition, an auxiliary such as dimethyl sulfoxide is added thereto for use, if desired, for enhancing fusion efficiency.

The ratio between the immunocytes and the myeloma cells used can be arbitrarily set. For example, the amount of the immunocytes is preferably set to 1 to 10 times the amount of the myeloma cells. For example, an RPMI1640 medium or a MEM medium suitable for the growth of the myeloma cell line as well as a usual medium for use in this kind of cell culture is used as the medium for use in the cell fusion. Preferably, a solution supplemented with serum (e.g., fetal calf serum (FCS)) can be further added to the medium.

For the cell fusion, the immunocytes and the myeloma cells are well mixed in the predetermined amounts in the medium. A PEG solution (e.g., average molecular weight: on the order of 1000 to 6000) preheated to approximately 37°C is usually added thereto at a concentration of 30 to 60% (w/v). The mixed solution is gently mixed so that desired fusion cells (hybridomas) are formed. Subsequently, the appropriate medium listed above is sequentially added to the cell cultures, and its supernatant is removed by centrifugation. This operation can be repeated to remove the cell fusion agents or the like unfavorable for hybridoma growth.

The hybridomas thus obtained can be cultured in a usual selective medium, for example, a HAT medium (medium containing hypoxanthine, aminopterin, and thymidine), for selection. The culture using the HAT medium can be continued for a time long enough to kill cells (non-fused cells) other than the desired hybridomas (usually, the time long enough is several days to several weeks). Subsequently, hybridomas producing the desired antibody are screened for and single-cell cloned by a usual limiting dilution method.

The hybridomas thus obtained can be selected by use of a selective medium appropriate for the selection marker of the myeloma used in the cell fusion. For example, the cells having the HGPRT or TK deficiency can be selected by culture in a HAT medium (medium containing hypoxanthine, aminopterin, and thymidine). Specifically, when HAT-sensitive myeloma cells are used in the cell fusion, only cells successfully fused with normal cells can be grown selectively in the HAT medium. The culture using the HAT medium is continued for a time long enough to kill cells (non-fused cells) other than the desired hybridomas. Specifically, the culture can generally be carried out for several days to several weeks to select the desired hybridomas. Subsequently, hybridomas producing the desired antibody can be screened for and single-cell cloned by a usual limiting dilution method.

The screening of the desired antibody and the single-cell cloning can be preferably carried out by a screening method based on antigen-antibody reaction known in the art. For example, the antibody can be evaluated for its binding activity against labeled protein C on the basis of the principle of ELISA. The protein C is immobilized onto each well of, for example, an ELISA plate. The hybridoma culture supernatant is contacted with the immobilized protein in the well to detect an antibody binding to the immobilized protein. In the case of a mouse-derived monoclonal antibody, the antibody bound with the cell can be detected using an anti-mouse immunoglobulin antibody. Hybridomas producing the desired antibody having the ability to bind to the antigen, thus selected by screening, can be cloned by a limiting dilution method or the like.

The monoclonal antibody-producing hybridomas thus prepared can be subcultured in a usual medium. The hybridomas can also be stored over a long period in liquid nitrogen.

The hybridomas are cultured according to a usual method, and the desired monoclonal antibody can be obtained from the culture supernatant thereof. Alternatively, the hybridomas may be administered to mammals compatible therewith and grown, and the monoclonal antibody can be obtained from the ascitic fluids thereof. The former method is suitable for obtaining highly pure antibodies.

An antibody encoded by an antibody gene cloned from the antibody-producing cells such as hybridomas may also be preferably used. The cloned antibody gene is incorporated in appropriate vectors, which are then transferred to hosts so that the antibody encoded by the gene is expressed. Methods for the antibody gene isolation, the incorporation into vectors, and the transformation of host cells have already been established by, for example, Vandamme et al. (Eur. J. Biochem. (1990) 192 (3), 767-775). A method for producing a recombinant antibody as mentioned below is also known in the art.

For example, cDNAs encoding the variable regions (V regions) of the anti-protein C antibody are obtained from the hybridoma cells producing the anti-protein C antibody. For this purpose, usually, total RNA is first extracted from the hybridomas. For example, the following methods can be used as a method for mRNA extraction from the cells:
- guanidine ultracentrifugation method (Biochemistry (1979) 18 (24), 5294-5299), and
- AGPC method (Anal. Biochem. (1987) 162 (1), 156-159).

The extracted mRNAs can be purified using mRNA Purification Kit (manufactured by GE Healthcare Bio-Sciences Corp.) or the like. Alternatively, a kit for directly extracting total mRNA from cells is also commercially available, such as QuickPrep mRNA Purification Kit (manufactured by GE Healthcare Bio-Sciences Corp.). The mRNAs may be obtained from the hybridomas using such a kit. From the obtained mRNAs, the cDNAs encoding antibody V regions can be synthesized using reverse transcriptase. The cDNAs can be synthesized using, for example, AMV Reverse Transcriptase First-strand cDNA Synthesis Kit (manufactured by Seikagaku Corp.). Alternatively, a 5'-RACE method (Proc. Natl. Acad. Sci. USA (1988) 85 (23), 8998-9002; and Nucleic Acids Res. (1989) 17 (8), 2919-2932) using SMART RACE cDNA amplification kit (manufactured by Clontech Laboratories, Inc.) and PCR may be appropriately used for the cDNA synthesis and amplification. In the course of such cDNA synthesis, appropriate restriction sites mentioned later can be further introduced into both ends of the cDNAs.

The cDNA fragments of interest are purified from the obtained PCR products and subsequently ligated with vector DNAs. The recombinant vectors thus prepared are transferred to *E. coli* or the like. After colony selection, desired recombinant vectors can be prepared from the *E. coli* that has formed the colony. Then, whether or not the recombinant vectors have the nucleotide sequences of the cDNAs of interest is confirmed by a method known in the art, for example, a dideoxynucleotide chain termination method.

The 5'-RACE method using primers for variable region gene amplification is conveniently used for obtaining the genes encoding variable regions. First, cDNAs are synthesized with RNAs extracted from the hybridoma cells as templates to obtain a 5'-RACE cDNA library. A commercially available kit such as SMART RACE cDNA amplification kit is appropriately used in the synthesis of the 5'-RACE cDNA library.

The antibody gene is amplified by PCR with the obtained 5'-RACE cDNA library as a template. Primers for mouse antibody gene amplification can be designed on the basis of an antibody gene sequence known in the art. These primers have nucleotide sequences differing depending on immunoglobulin subclasses. Thus, the subclass is desirably determined in advance using a commercially available kit such as Iso Strip mouse monoclonal antibody isotyping kit (Roche Diagnostics K.K.).

Specifically, primers capable of amplifying genes encoding γ1, γ2a, γ2b, and γ3 heavy chains and κ and λ light chains can be used, for example, for the purpose of obtaining a gene encoding mouse IgG. Primers that anneal to portions corresponding to constant regions close to variable regions are generally used as 3' primers for amplifying IgG variable region genes. On the other hand, primers included in the 5' RACE cDNA library preparation kit are used as 5' primers.

The PCR products thus obtained by amplification can be used to reshape an immunoglobulin composed of heavy chains and light chains in combination. The desired antibody can be screened for with the binding activity of the reshaped immunoglobulin against protein C as an index. More preferably, the binding of the antibody to protein C is specific for the purpose of obtaining the antibody against protein C. The antibody binding to protein C can be screened for, for example, by the following steps:
(1) contacting each antibody comprising V regions encoded by the cDNAs obtained from the hybridomas, with protein C;
(2) detecting the binding between the protein C and the antibody; and
(3) selecting the antibody binding to protein C.

A method for detecting the binding between the antibody and the protein C is known in the art. Specifically, the binding between the antibody and the protein C can be detected by an approach such as ELISA mentioned above. A fixed preparation of protein C can be appropriately used for evaluating the binding activity of the antibody.

A panning method using phage vectors is also preferably used as a method for screening for the antibody with its binding activity as an index. When antibody genes are obtained as libraries of heavy chain and light chain subclasses from a polyclonal antibody-expressing cell population, a screening method using phage vectors is advantageous. Genes encoding heavy chain and light chain variable regions can be linked via an appropriate linker sequence to form a gene encoding single-chain Fv (scFv). The gene encoding scFv can be inserted to phage vectors to obtain phages expressing scFv on their surface. The phages thus obtained are contacted with the desired antigen. Then, antigen-bound phages can be recovered to recover a DNA encoding scFv having the binding activity of interest. This operation can be repeated, if necessary, to enrich scFvs having the desired binding activity.

After the obtainment of the cDNA encoding each V region of the anti-protein C antibody of interest, this cDNA is digested with restriction enzymes that recognize the restriction sites inserted in both ends of the cDNA. The restriction enzymes preferably recognize and digest a nucleotide sequence that appears low frequently in the nucleotide sequence constituting the antibody gene. The insertion of restriction enzymes that provide cohesive ends is preferred for inserting one copy of the digested fragment in the correct direction in a vector. The thus-digested cDNAs encoding the V regions of the anti-protein C antibody can be inserted to appropriate expression vectors to obtain antibody expression vectors. In this case, genes encoding antibody constant regions (C regions) and the genes encoding the V regions are fused in frame to obtain a chimeric antibody. In this context, the "chimeric antibody" refers to an antibody comprising constant and variable regions of different origins. Thus, heterogeneous (e.g., mouse-human) chimeric antibodies as well as human-human homogeneous chimeric antibodies are also encompassed by the chimeric antibody according to the present invention. The V region genes can be inserted to expression vectors preliminarily having constant region genes to construct chimeric antibody expression vectors. Specifically, for example, recognition sequences for restriction enzymes that digest the V region genes can be appropriately located on the 5' side of an expression vector carrying the DNAs encoding the desired antibody constant regions (C regions). This expression vector having the C region genes and the V region genes are digested with the same combination of restriction enzymes and fused in frame to construct a chimeric antibody expression vector.

The isotype of each antibody depends on the structure of its constant regions. The constant regions of antibodies of isotypes IgG1, IgG2, IgG3, and IgG4 are called Cγ1, Cγ2, Cγ3, and Cγ4, respectively. The constant regions of λ and κ chains are appropriately used as the light chain constant regions of the antibody.

In order to produce the monoclonal antibody binding to protein C, the antibody gene is incorporated into expression vectors such that the antibody gene is expressed under the control of expression control regions. The expression control regions for antibody expression include, for example, an enhancer and a promoter. Also, an appropriate signal sequence can be added to the amino terminus such that the expressed antibody is extracellularly secreted. The expressed polypeptide is cleaved at the carboxyl terminal moiety of this sequence. The cleaved polypeptide can be extracellularly secreted as a mature polypeptide. Furthermore, appropriate host cells can be transformed with these expression vectors to obtain recombinant cells expressing the DNA encoding the anti-protein C antibody.

For the antibody gene expression, DNAs encoding the heavy chain (H chain) and the light chain (L chain) of the antibody are separately incorporated into different expression vectors. The same host cell can be co-transfected with these vectors carrying the H chain gene and the L chain gene and thereby allowed to express an antibody molecule comprising the H chain and the L chain. Alternatively, the DNAs encoding the H chain and L chain may be incorporated into a single expression vector, with which host cells can be transformed (see WO1994/011523).

Many combinations of host cells and expression vectors are known in the art for preparing the antibody by transferring the isolated antibody gene into appropriate hosts. All of these expression systems can be applied to the isolation of the antigen-binding domain of the present invention. In the case of using eukaryotic cells as the host cells, animal cells, plant cells, or fungus cells can be appropriately used. Specifically, examples of the animal cells can include the following cells:
(1) mammalian cells such as CHO, COS, myeloma, BHK (baby hamster kidney), Hela, Vero, and HEK (human embryonic kidney) 293;
(2) amphibian cells such as *Xenopus* oocytes; and
(3) insect cells such as sf9, sf21, and Tn5.

Alternatively, antibody gene expression systems using cells derived from the genus *Nicotiana* (e.g., *Nicotiana tabacum*) as the plant cells are known in the art. Cultured callus cells can be appropriately used for the plant cell transformation.

The following cells can be used as the fungus cells:
cells derived from yeasts of the genus *Saccharomyces* (e.g., *Saccharormyces cerevisiae)* and the genus *Pichia* (e.g., *Pichia pastoris*), and
cells derived from filamentous fungi of the genus *Aspergillus* (e.g., *Aspergillus niger*).

Antibody gene expression systems using prokaryotic cells are also known in the art. In the case of using, for example, bacterial cells, cells of bacteria such as *E. coli* and *Bacillus subtilis* can be appropriately used. The expression vectors containing the antibody gene of interest are transferred into these cells by transformation. The transformed cells are cultured *in vitro*, and the desired antibody can be obtained from the cultures of the transformed cells.

In addition to the host cells, transgenic animals may be used for the production of the recombinant antibody. Specifically, the desired antibody can be obtained from animals transfected with the gene encoding this antibody. For example, the antibody gene can be inserted in frame into a gene encoding a protein specifically produced in milk to thereby construct a fusion gene. For example, goat β casein can be used as the protein secreted into milk. A DNA fragment containing the fusion gene having the antibody gene insert is injected into goat embryos, which are in turn introduced into female goats. From milk produced by transgenic goats (or progeny thereof) brought forth by the goats that have received the embryos, the desired antibody can be obtained as a fusion protein with the milk protein. In order to increase the amount of milk containing the desired antibody produced from the transgenic goats, hormone can be administered to the transgenic goats (Bio/Technology (1994) 12 (7), 699-702).

In the case of administering the anti-LAMP5 antibody described in the present specification to humans, an antigen-binding domain derived from a genetically recombinant antibody that has been engineered artificially can be appropriately adopted as the antigen-binding domain in the antibody, for example, for the purpose of reducing heteroantigenicity in humans. The genetically recombinant antibody encompasses, for example, humanized antibodies. Such an engineered antibody is appropriately produced using a method known in the art.

Each antibody variable region that is used for preparing the antigen-binding domain in the anti-protein C antibody of the present invention is usually constituted by three complementarity-determining regions (CDRs) flanked by four framework regions (FRs). The CDRs are regions that substantially determine the binding specificity of the antibody. The CDRs have highly diverse amino acid sequences. On the other hand, the amino acid sequences constituting the FRs often exhibit high identity even among antibodies differing in binding specificity. Therefore, in general, the binding specificity of an antibody can reportedly be transplanted to another antibody by CDR grafting.

The humanized antibody is also called reshaped human antibody. Specifically, for example, a humanized antibody comprising non-human animal (e.g., mouse) antibody CDRs grafted in a human antibody is known in the art. General gene recombination approaches are also known for obtaining the humanized antibody. Specifically, for example, overlap extension PCR is known in the art as a method for grafting mouse antibody CDRs to human FRs. In the overlap extension PCR, nucleotide sequences encoding mouse antibody CDRs to be grafted are added to primers for human antibody FR synthesis. The primers are prepared for each of the four FRs. In the mouse CDR grafting to the human FRs, in general, the selection of human FRs highly homologous to the mouse FRs is reportedly advantageous in maintaining the CDR functions. Specifically, in general, it is preferred to use human FRs comprising amino acid sequences highly identical to those of the mouse FRs adjacent to the mouse CDRs to be grafted.

The nucleotide sequences to be linked are designed such that the sequences are connected in frame. DNAs encoding human FRs are individually synthesized with their respective primers. The resulting PCR products contain the mouse CDR-encoding DNA added to each human FR-encoding DNA. The mouse CDR-encoding nucleotide sequences are designed such that the nucleotide sequence in each product overlaps with another. Subsequently, the overlapping CDR portions in the products synthesized with the human antibody gene as a template are annealed to each other for complementary strand synthesis reaction. Through this reaction, the human FR sequences are linked via the mouse CDR sequences.

Finally, the full-length gene of the V region comprising three CDRs and four FRs thus linked is amplified using primers that respectively anneal to the 5' and 3' ends thereof and have the added recognition sequences for appropriate restriction enzymes. The DNA thus obtained and the DNA encoding the human antibody C region can be inserted into expression vectors such that these DNAs are fused in frame to prepare vectors for human-type antibody expression.

These vectors carrying the DNAs are transferred to hosts to establish recombinant cells. Then, the recombinant cells are cultured for the expression of the DNA encoding the humanized antibody to produce the humanized antibody into the cultures of the cultured cells (see European Patent Publication No. EP239400 and International Publication No. WO1996/002576).

The humanized antibody thus prepared can be evaluated for its binding activity against the antigen by qualitative or quantitative assay to thereby select suitable human antibody FRs that allow the CDRs to form a favorable antigen-binding site when linked via the CDRs. If necessary, FR amino acid residue(s) may be substituted such that the CDRs of the resulting reshaped human antibody form an appropriate antigen-binding site. For example, a mutation can be introduced in the amino acid sequence of human FR by the application of the PCR method used in the mouse CDR grafting to the human FRs. Specifically, a mutation of a partial nucleotide sequence can be introduced to the primers annealing to a FR nucleotide sequence. The FR nucleotide sequence synthesized using such primers contains the mutation thus introduced. The variant antibody having the substituted amino acid(s) can be evaluated for its binding activity against the antigen by assay in the same way as above to thereby select variant FR sequences having the desired properties (Cancer Res., (1993) 53, 851-856).

Furthermore, transgenic animals having all repertoires of human antibody genes (see International Publication Nos. WO1993/012227, WO1992/003918, WO1994/002602, WO1994/025585, WO1996/034096, and WO1996/033735) can be used as animals to be immunized by DNA immunization to obtain the desired human antibody.

In addition, a technique of obtaining a human antibody by panning using a human antibody library is also known. For example, human antibody V regions are expressed as a single-chain antibody (scFv) on the surface of phages by a phage display method. A phage expressing scFv binding to the antigen can be selected. The gene of the selected phage can be analyzed to determine DNA sequences encoding the V regions of the human antibody binding to the antigen. After the determination of the DNA sequence of the scFv binding to the antigen, the V region sequences are fused in frame with the sequences of the desired human antibody C regions. Then, this fusion product can be inserted to appropriate expression vectors to prepare expression vectors. The expression vectors are transferred to the suitable expression cells as listed above. The cells are allowed to express the gene encoding the human antibody to obtain the human antibody. These methods have already been known in the art (see International Publication Nos. WO1992/001047, WO1992/020791, WO1993/006213, WO1993/011236, WO1993/019172, WO1995/001438, and WO1995/015388).

In addition to the methods described above, an approach of B cell cloning (identification and cloning of the coding sequence of each antibody, isolation thereof, and use for expression vector construction for the preparation of each antibody (particularly, IgG1, IgG2, IgG3, or IgG4), etc.) as described in Bernasconi et al. (Science (2002) 298, 2199-2202) or WO2008/081008 can be appropriately used as a method for obtaining the antibody gene.

Whether to inhibit the activation of protein C can be confirmed according to a method known in the art. For example, a candidate agent that may inhibit the activation of protein C is contacted with protein C. Then, thrombin and thrombomodulin are added thereto to start thrombin-mediated protein C activation reaction. Then, the activation reaction of the protein C is terminated. The activity of the protein C activated by the thrombin can be measured to confirm whether the candidate agent inhibits the activation of protein C. Specific procedures are as described in Example 4.

The agent inhibiting the activation of protein C, obtained by the method described above, can be formulated as a pharmaceutical composition according to a routine method.

The pharmaceutical composition of the present invention may be further used in combination with an agent inhibiting the activity of the activated protein C. Alternatively, the agent inhibiting the activation of protein C may also have the activity of inhibiting the activation of the activated protein C.

In the present invention, the phrase "inhibition of the activity of the activated protein C" or "inhibiting the activity of the activated protein C" means to inhibit the inactivation of activated coagulation factor V or activated coagulation factor VIII by the activated protein C. Examples of the agent inhibiting the activity of the activated protein C include agents inhibiting the binding of the activated protein C to activated coagulation factor V or activated coagulation factor VIII. Specific examples thereof include anti-activated protein C antibodies, anti-activated coagulation factor V antibodies, anti-activated coagulation factor VIII antibodies, partial peptides of activated protein C, partial peptides of activated coagulation factor V, partial peptides of activated coagulation factor VIII, and low-molecular compounds that exhibit similar activity thereto.

In the present invention, the phrase "used in combination with an agent inhibiting the activity of the activated protein C" means that the agent inhibiting the activation of protein C and the agent inhibiting the activity of the activated protein C are combined for simultaneous, separate, or sequential administration. The agent inhibiting the activation of protein C and the agent inhibiting the activity of the activated protein C may contained in one pharmaceutical composition or may be contained in separate pharmaceutical compositions. Alternatively, these agents may be constituted as a kit containing a pharmaceutical composition comprising the agent inhibiting the activation of protein C and a pharmaceutical composition comprising the agent inhibiting the activity of the activated protein C.

When the agent inhibiting the activation of protein C also inhibits the activity of the activated protein C, examples of such an agent include antibodies. Specifically, among the anti-protein C antibodies mentioned above, preferred examples of such an agent can include antibodies further having an effect of inhibiting the activity of the activated protein C. More specific examples thereof include antibodies binding to both protein C and activated protein C.

Whether to inhibit the activity of the activated protein C can be confirmed according to a method known in the art. For example, a candidate agent that may inhibit the activity of the activated protein C is contacted with activated protein C. Then, activated coagulation factor V is added thereto to start the activated protein C-mediated inactivation reaction of the activated coagulation factor V. Then, activated blood coagulation factor X and prothrombin are added thereto to start thrombin generation dependent on the activated coagulation factor V. The activity of the generated thrombin can be measured to confirm whether the candidate agent inhibits the inactivation by the activated protein C. Specific procedures are as described in Example 3.

The pharmaceutical composition according to the present invention can be used for inhibiting the anticoagulant effect of the activated protein C or for promoting a hemostatic effect, because the contained agent inhibiting the activation of protein C has effects of inhibiting the anticoagulant effect of the activated protein C and promoting blood coagulation. By virtue of this effect of promoting blood coagulation, the pharmaceutical composition according to the present invention can be used in the treatment of a hemorrhagic disease caused by reduction in blood-clotting function. Examples of such a disease can include bleeding, diseases involving bleeding, and diseases caused by bleeding. Specific examples thereof include hemophilia, acquired hemophilia, von Willebrand disease caused by functional abnormality or deficiency in von Willebrand factor (vWF), and acquired von Willebrand disease.

Thus, the present invention provides a pharmaceutical composition for the inhibition of an anticoagulant effect, a pharmaceutical composition for the promotion of a hemostatic effect, or a pharmaceutical composition for the treatment of a hemorrhagic disease, comprising the agent inhibiting the activation of protein C.

The pharmaceutical composition of the present invention can be administered either orally or parenterally to a patient. Parenteral administration is preferred. Specific examples of such an administration method include injection administration, transnasal administration, transpulmonary administration, and transdermal administration. Examples of the injection administration include intravenous injection, intramuscular injection, intraperitoneal injection, and subcutaneous injection, through which the pharmaceutical composition of the present invention can be administered systemically or locally. The administration method can be appropriately selected according to the age or symptoms of the patient. The dose can be selected from among the range of, for example, 0.0001 mg to 1000 mg per kg body weight per dosing. Alternatively, the dose for each patient can be selected from among the range of, for example, 0.001 to 100000 mg per body. However, the pharmaceutical composition of the present invention is not limited by these doses.

The pharmaceutical composition of the present invention can be formulated according to a routine method (e.g., Remington's Pharmaceutical Science, latest edition, Mark Publishing Company, Easton, U.S.A.). The pharmaceutical composition may additionally contain pharmaceutically acceptable carriers or additives. Examples of the pharmaceutically acceptable carriers or additives include surfactants, excipients, coloring agents, flavoring agents, preservatives, stabilizers, buffers, suspending agents, tonicity agents, binders, disintegrants, lubricants, flow promoters, and corrigents. The pharmaceutically acceptable carriers or additives according to the present invention are no limited to them, and other carriers or additives routinely used can be appropriately used. Specific examples of the carriers can include light anhydrous silicic acid, lactose, crystalline cellulose, mannitol, starch, carmellose calcium, carmellose sodium, hydroxypropylcellulose, hydroxypropylmethylcellulose, polyvinyl acetal diethylaminoacetate, polyvinylpyrrolidone, gelatin, medium-chain fatty acid triglyceride, polyoxyethylene hydrogenated castor oil 60, white sugar, carboxymethylcellulose, corn starch, and inorganic salts.

If necessary, the antibody of the present invention can be enclosed in microcapsules (e.g., hydroxymethylcellulose, gelatin, and poly[methyl methacrylate] microcapsules) or can also be prepared as a colloidal drug delivery system (e.g., liposomes, albumin microspheres, microemulsions, nanoparticles, and nanocapsules) (see e.g., "Remington's Pharmaceutical Science 16th edition", Oslo Ed. (1980)). A method for preparing drugs as sustained-release drugs is also known in the art and can be applied to the antibody of the present invention (Langer et al., J. Biomed. Mater. Res. 15: 267-277 (1981); Langer, Chemtech. 12: 98-105 (1982); U.S. Patent No. 3,773,919; European Patent Application Publication No. EP58481; Sidman et al., Biopolymers 22: 547-556 (1983); and EP133988).

The present invention also provides a method for preventing and/or treating bleeding, a disease involving bleeding, or a disease caused by bleeding, comprising the step of administering the antibody or the composition of the present invention. The administration of the antibody or the composition can be carried out by, for example, any of the methods described above.

The present invention further provides a kit for use in the method described above, comprising at least the antibody or the composition of the present invention. In the kit, for example, a syringe, an injection needle, a pharmaceutically acceptable vehicle, alcohol cotton, a plaster, or an instruction stating the usage can also be additionally packaged.

The present invention also relates to use of the pharmaceutical composition of the present invention comprising the agent inhibiting the activation of protein C, for the production of a preventive and/or therapeutic agent for bleeding, a disease involving bleeding, or a disease caused by bleeding.

The present invention also relates to the multispecific antigen-binding molecule or the bispecific antibody, or the composition of the present invention for the prevention and/or treatment of bleeding, a disease involving bleeding, or a disease caused by bleeding.

All prior technical literatures cited herein are incorporated herein by reference.

### Examples

The present invention will be described further specifically with reference to Examples. However, the present invention is not intended to be limited by these examples. Those skilled in the art can make various changes or modifications in the present invention. These changes or modifications are also included in the scope of the present invention.

### [Example 1] Preparation of mouse protein C

A cDNA spanning the full-length coding region of mouse protein C was amplified by PCR from the mouse liver. A "gene" encoding C-terminally FLAG-tagged protein C was further amplified by "PCR" with this cDNA as a template and subcloned into expression vectors. CHO cells were transfected with the expression vectors and cultured. The mouse protein C-Flag protein was purified from the obtained culture supernatant according to a routine method.

### [Example 2] Preparation of anti-mouse protein C rat antibody

The mouse protein C-Flag protein (0.1 mg/head) prepared in Example 1 was mixed with a Freund's complete adjuvant (FCA, Difco Laboratories Inc., currently Becton, Dickinson and Company). The mixture was inoculated to the foot pad of one leg of each SD rat (two individuals, Charles River Laboratories Japan, Inc.). Two weeks after the inoculation, the iliac lymph node was excised from the immunized rat. Hybridomas were prepared according to a routine method using the excised lymph node cells. The binding activity of an antibody produced by each hybridoma against the mouse protein C-Flag and the Flag protein was measured by ELISA using the culture supernatants of the hybridomas. Hybridomas producing antibodies specifically exhibiting binding activity against the mouse protein C were selected. The selected hybridomas were cultured. Anti-mouse protein C antibodies were purified from the culture supernatants according to a routine method. The purified antibodies were screened by methods described in Examples 3 and 4, etc., to select MP35 and MP51 as antibodies inhibiting the activation of the mouse protein C and/or the activity of the activated protein C.

### [Example 3] Effects of anti-mouse protein C antibodies MP35 and MP51 on inactivation of FVa by mouse activated protein C

### (Method)

### (1) Preparation of reagent

Each anti-mouse protein C antibody was adjusted to 15 and 50 µg/mL with tris-buffered saline containing 0.1% bovine serum albumin (TBSB).

Hirudin (Merck KGaA) was adjusted to 10 IU/mL with TBSB.

Mouse protein C-Flag, human α thrombin (Enzyme Research Laboratories Inc.), rabbit thrombomodulin (American Diagnostica Inc.), human activated coagulation factor X (FXa, Enzyme Research Laboratories Inc.), human activated coagulation factor V (FVa, Enzyme Research Laboratories Inc.), and human prothrombin (Enzyme Research Laboratories Inc.) were adjusted to 24.8 µg/mL, 1.48 µg/mL, 14.8 µg/mL, 9.66 ng/mL, 2.25 ng/mL, and 50 µg/mL, respectively, with TBSB containing a 104 µM phospholipid solution (10% phosphatidylserine/60% phosphatidylcholine/30% phosphatidylethanolamine (Avanti Polar Lipids); prepared according to Okuda, M. & Yamamoto, Y. Clin. Lab. Haem. 26, 215-223 (2004)), 8.3 mM CaCl₂, and 1.7 mM MgCl₂ (TBCP).

Mouse activated protein C was prepared by mixing equal amounts of 24.8 µg/mL mouse protein C-Flag, 1.48 µg/mL human α thrombin, and 14.8 µg/mL rabbit thrombomodulin, incubating the mixture at 37°C for 120 minutes, and then adding 10 U/mL hirudin in an amount of 1/3 of the volume of the mixed solution. This protein was diluted 3333-fold with TBSB.

S-2238 (CHROMOGENIX) was dissolved at 4 mM in purified water and then further diluted 1.6-fold with purified water.

### (2) Assay

In each well of a 96-well plate, 5 µL of the mouse activated protein C and 5 µL of 0.5, 1.5, 5, 15, 50, 150, or 500 µg/mL anti-mouse protein C antibody prepared in the paragraph (1) were mixed and incubated at room temperature for 30 minutes (for a group not supplemented with the antibody, the protein was mixed with 5 µL of TBSB instead of the antibody solution; and for a group supplemented with neither the mouse activated protein C nor the antibody, 5 µL of TBCP and 5 µL of TBSB were mixed instead of them).

Subsequently, 5 µL of 2.25 ng/mL FVa was added thereto at room temperature to start FVa inactivation reaction. In order to evaluate the activity of residual FVa, 5 µL of 9.66 ng/mL human FXa and 5 µL of 50 µg/mL human prothrombin were added thereto 15 minutes later to start thrombin generation reaction dependent on the FVa concentration. After 10 minutes, the thrombin generation reaction was terminated by the addition of 5 µL of 0.5 M EDTA. In order to measure the activity of the generated thrombin, 5 µL of the chromogenic substrate solution S-2238 was added thereto to start color reaction. After the 15-minute color reaction, change in absorbance at 405 nm was measured using SpectraMax 340PC³⁸⁴ (Molecular Devices, LLC).

### (Results)

Both of the anti-mouse protein C antibodies MP35 and MP51 elevated the absorbance (Figure 1). These results demonstrated that both of MP35 and MP51 inhibit the inactivation of FVa by mouse activated protein C.

### [Example 4] Effects of anti-mouse protein C antibodies MP35 and MP51 on activation of mouse protein C by thrombin/thrombomodulin complex

### (Method)

### (1) Preparation of reagent

Each anti-mouse protein C antibody and hirudin were adjusted to 120 µg/mL and 75 U/mL, respectively, with TBSB.

Mouse protein C-Flag, human α thrombin, and rabbit thrombomodulin were adjusted to 24.8 µg/mL, 14.8 µg/mL, and 14.8 µg/mL, respectively, with TBCP.

Spectrozyme aPC (American Diagnostica Inc.) was dissolved at 5 mM in purified water and then further diluted 6.25-fold with purified water.

### (2) Assay

In each well of a 96-well plate, 5 µL of 120 µg/mL anti-mouse protein C antibody and 5 µL of 24.8 µg/mL mouse protein C-Flag protein solution were mixed and incubated at room temperature for 30 minutes (for a group not supplemented with the antibody, the protein was mixed with 5 µL of TBSB instead of the antibody solution).

Subsequently, 5 µL of 14.8 µg/mL human α thrombin and 5 µL of 14.8 µg/mL rabbit thrombomodulin were added thereto at 37°C to start mouse protein C activation reaction. After 120 minutes, the protein C activation reaction was terminated by the addition of 10 µL of 75 U/mL hirudin. In order to measure the activity of the resulting activated protein C, 10 µL of the chromogenic substrate solution Spectrozyme aPC was added thereto to start color reaction. After the 45-minute color reaction, change in absorbance at 405 nm was measured using an absorption spectrometer SpectraMax 340PC³⁸⁴ (Molecular Devices, LLC).

### (Results)

The absorbance was decreased by the addition of the anti-mouse protein C antibody MP51. By contrast, the absorbance exhibited no change even by the addition of MP35 (Figure 2). These results demonstrated that MP51 inhibits protein C activation reaction, whereas MP35 does not inhibit this reaction. The antibody concentration in the mouse protein C activation reaction was 40 µg/mL.

### [Example 5] Effect of anti-mouse protein C antibody on thrombin generation in mouse plasma supplemented with agent activating protein C

### (Method)

### (1) Preparation of reagent

Each anti-mouse protein C antibody was adjusted to 0.18, 0.6, 1.8, 6, 18, 60, and 180 µg/mL with TBSB.

An agent activating protein C (trade name: Protac, American Diagnostica Inc.) was dissolved in purified water (6 U/mL) and further adjusted to 4 U/mL with TBS.

### (2) Assay

In each well of a 96-well plate for fluorescent assay (Thermo Fisher Scientific K.K., Immulon 2HB "U" Bottom Microtiter Plates, 3655), 25 µL of 0.18, 0.6, 1.8, 6, 18, 60, or 180 µg/mL anti-mouse protein C antibody and 15 µL of mouse citrate plasma were mixed, and the plate was left standing at room temperature for 15 minutes (for a group not supplemented with the antibody, the plasma was mixed with 5 µL of TBSB instead of the antibody solution).

Subsequently, 40 µL of 4 U/mL agent activating protein C (trade name: Protac, American Diagnostica Inc.) and 20 µL of a coagulation-initiating reagent PPP-Reagent LOW (Thrombinoscope B.V.) were added thereto, and the plate was left standing at 37°C for 15 minutes at room temperature.

In order to measure thrombin generation without activating protein C by Protac, a sample supplemented with the same volume of TBSB as that of the antibody solution instead thereof and the same volume of TBS as that of Protac instead thereof was also prepared.

Then, the plate was loaded in a thrombin generation fluorescence system and incubated at 37°C for approximately 5 minutes, followed by the start of the assay (at the start of the assay, 20 µL of a mixed solution of Fluo-Substrate and Fluo-Buffer was added).

In order to convert fluorescence intensity obtained from each sample to the amount of thrombin, a well was also established in the same plate by adding 20 µL of Thrombin Calibrator (Thrombinoscope B.V.) instead of the coagulation-initiating reagent to a mixed solution of 15 µL of mouse plasma and 25 µL of TBSB.

The conditions of Thrombinoscope software version 3.0.0.29 (Thrombinoscope B.V.), which is analysis software dedicated for the thrombin generation fluorescence system, were set as follows:
Amount of the mixed solution of Fluo-Substrate and Fluo-Buffer (setting: Dispense): 20 µL
Stirring time (setting item: Shake): 10 seconds
Measurement time (setting item: Total time): 60 minutes
Measurement interval (setting item: Interval): 20 seconds
Excitation wavelength (automatic setting): 390 nm
Fluorescence wavelength (automatic setting): 460 nm

The calculated starting time of thrombin generation (lag time (min)) and the largest amount of thrombin generated (peak height (nmol/L)) were used to evaluate the ability of the anti-mouse protein C antibody to inhibit the activity of the activated protein C in the mouse plasma.

### (Results)

Figure 3A shows the effect of each anti-mouse protein C antibody on thrombin generation in normal mouse plasma supplemented with the agent activating protein C (Protac). As a result of adding Protac to the normal mouse plasma, the lag time was prolonged from 2.0 minutes to 2.4 minutes, and the peak height was decreased from 53 nM to 22 nM. The activated protein C generated by Protac was thus confirmed to exhibit an anti-clotting effect in this plasma. Both of the anti-mouse protein C antibodies MP35 and MP51 shortened the lag time and increased the peak height in a dose-dependent manner in the presence of Protac.

FVIII-deficient mouse plasma was also evaluated in the same way as above (Figure 3B). As a result of adding Protac to the FVIII-deficient mouse plasma, the lag time was prolonged from 2.2 minutes to 2.5 minutes, and the peak height was decreased from 33 nM to 15 nM. The activated protein C generated by Protac was thus confirmed to exhibit an anti-clotting effect in this plasma. Both of MP35 and MP51 shortened the lag time and increased the peak height in a concentration-dependent manner in the presence of Protac.

These results demonstrated that MP35 and MP51 have the potential for promoting a coagulation effect by inhibiting the anti-clotting effect of the activated protein C in the normal mouse plasma or the FVIII-deficient mouse plasma.

As for inhibitory activity against the mouse activated protein C in the plasma, MP35 was found to have the activity equivalent to or higher than that of MP51.

### [Example 6] Production of FVIII-deficient nude mouse

An FVIII-deficient mouse (B6; 129S4-F8^{tm1Kaz/J mice}) was mated with a nude mouse (Crlj: CD1-Foxn1^{nu}) to introduce a hairless phenotype into the FVIII-deficient mouse.

### [Example 7] Effect of anti-mouse protein C antibody on bleeding symptom in puncture bleeding model using FVIII-deficient nude mouse.

### (Method)

A vehicle (n = 8), 3 mg/kg anti-mouse protein C antibody MP51 (n = 9), or 30 mg/kg anti-mouse protein C antibody MP35 (n = 9) was intravenously administered to the tail of each hemophilia A mouse. Two sites in the right and left medial thigh muscles of the mouse were punctured at a depth of 3 mm with a 23 G injection needle under isoflurane anesthesia. The puncture date was defined as Day 0. Bleeding areas visible from the body surface were measured at Day 1 and Day 2. The respective bleeding areas measured at Day 1 and Day 2 were summated for each mouse individual to determine a total bleeding area, which was used as an index for bleeding.

### (Results)

In the vehicle group, the total bleeding area was increased by bleeding induced by puncture, as the time passed. In the group given the anti-mouse protein C antibody MP51 (3 mg/kg) inhibiting the activation of protein C and the activity of the activated protein C, such increase in total bleeding area was prevented at both of Day 1 and Day 2. On the other hand, in the group given MP35 (30 mg/kg) inhibiting only the activity of the activated protein C, the total bleeding area was increased, as in the vehicle group (Figure 4). These results demonstrated that the anti-protein C antibody inhibiting the activation of protein C and the activity of the activated protein C has a hemostatic effect on a bleeding symptom in hemophilia A.

### [Example 8] Analysis of antigen-binding site in anti-mouse protein C antibody

### (Method)

MP35 and MP51 were each studied by Western blotting (WB) for whether to recognize the light chain or the heavy chain of mouse PC. Mouse PC ([trade name] Recombinant Mouse Coagulation Factor XIV/Protein C, [distributor] R&D Systems, Inc. [catalog No.] 4885-SE) was activated with human thrombin ([trade name] Human alpha Thrombin, [distributor] Enzyme Research Laboratories Inc., [catalog No.] HT 1002a) and rabbit thrombomodulin ([trade name] Rabbit Thrombomodulin, [distributor] Haematologic Technologies Inc., [catalog No.] RTM-2020). The resulting mouse activated PC was subjected to SDS-PAGE, then transferred to a PVDF membrane, and reacted with MP35 or MP51. A protein binding to MP35 or MP51 was detected using a secondary antibody ([trade name] HRP-Goat anti-Rat IgG (H+L), [distributor] Life Technologies Corp., [catalog No.] 629520) and a substrate ([trade name] SuperSignal West Dura Extended Duration Substrate, [distributor] Thermo Fisher Scientific K.K., [catalog No.] 34076).

### (Results)

As a result of WB, MP35 was found to bind to a protein between 15 and 20 kDa. This protein was confirmed to be the light chain of mouse activated PC, from the molecular weight and the N-terminal sequence. On the other hand, MP51 was found to bind to a protein between 37 and 50 kDa. This protein was confirmed to be the heavy chain of mouse activated PC, from the molecular weight and the N-terminal sequence. These results demonstrated that MP35 recognizes the light chain of mouse PC or mouse activated PC, and MP51 recognizes the heavy chain thereof.

### [Example 9] Preparation of anti-mouse protein C human antibody

Display phages binding to mouse protein C were enriched from a human naive antibody library by the phage display method using the mouse protein C prepared in Example 1. Display phages exhibiting binding activity specific for the mouse protein C were selected, and antibody variable region genes were amplified therefrom. Genetically recombinant IgG was expressed from the genes according to a routine method and purified. The purified antibodies were screened by the methods described in Examples 3 and 4, etc., to select L2 and L12 as antibodies inhibiting the activation of the mouse protein C and/or the activity of the activated protein C.

### [Example 10] Effects of anti-mouse protein C antibodies L2 and L12 on inactivation of FVa by mouse activated protein C

### (Method)

The method followed the method of Example 3.

### (Results)

Both of the anti-mouse protein C antibodies L2 and L12 elevated the absorbance (Figure 5). These results demonstrated that both of L2 and L12 inhibit the inactivation of FVa by mouse activated protein C. The antibody concentration in the mouse protein C activation reaction was 15 or 50 µg/mL.

### [Example 11] Effects of anti-mouse protein C antibodies L2 and L12 on activation of mouse protein C by thrombin/thrombomodulin complex

### (Method)

The method followed the method of Example 4.

### (Results)

The absorbance was decreased by the addition of the anti-mouse protein C antibody L12. By contrast, the absorbance exhibited no change even by the addition of L2 (Figure 6). These results demonstrated that L12 inhibits protein C activation reaction, whereas L2 does not inhibit this reaction. The antibody concentration in the mouse protein C activation reaction was 30 µg/mL.

### Industrial Applicability

According to the present invention, the anticoagulant effect of protein C can be inhibited or suppressed, and a blood-clotting effect and/or a hemostatic effect can be promoted. Thus, the present invention has enabled a novel therapeutic drug or a novel treatment method to be developed for a hemorrhagic disease.

## Claims

1. A pharmaceutical composition for the treatment of a hemorrhagic disease, comprising an agent inhibiting the activation of protein C.

2. The composition according to claim 1, wherein the hemorrhagic disease is a disease selected from hemophilia, acquired hemophilia, von Willebrand disease caused by functional abnormality or deficiency in vWF, and acquired von Willebrand disease.

3. The composition according to claim 1, wherein the hemorrhagic disease is hemophilia A.

4. A pharmaceutical composition for the promotion of a hemostatic effect, comprising an agent inhibiting the activation of protein C.

5. The composition according to claim 4, wherein the hemostatic effect is an effect on the bleeding symptom of a disease selected from hemophilia, acquired hemophilia, von Willebrand disease caused by functional abnormality or deficiency in vWF, and acquired von Willebrand disease.

6. The composition according to claim 4, wherein the hemostatic effect is an effect on the bleeding symptom of hemophilia A.

7. A pharmaceutical composition for the inhibition of an anticoagulant effect, comprising an agent inhibiting the activation of protein C.

8. The composition according to claim 7, wherein the anticoagulant effect is the anticoagulant effect of activated protein C.

9. The composition according to any one of claims 1 to 8, wherein the agent inhibiting the activation of protein C further inhibits the activity of the activated protein C.

10. The composition according to any one of claims 1 to 9, wherein the pharmaceutical composition is a composition that is used in combination with an agent inhibiting the activity of the activated protein C.

11. The composition according to any one of claims 1 to 10, wherein the agent inhibiting the activation of protein C is an anti-protein C antibody.

12. The composition according to claim 11, wherein the anti-protein C antibody is an antibody binding to the heavy chain of the protein C.

13. An anti-protein C antibody having an effect of inhibiting the conversion of protein C to activated protein C by binding to the heavy chain of the protein C.

14. The antibody according to claim 13, wherein the anti-protein C antibody further has an effect of inhibiting the activity of the activated protein C.
